(19) Europäisches Patentamt
European Patent Office
Office européen des brevets

(11) EP 0 697 867 B1

(12) EUROPÄISCHE PATENTSCHRIFT

(45) Veröffentlichungstag und Bekanntmachung des Hinweises auf die Patenterteilung:
**05.12.2001 Patentblatt 2001/49**

(21) Anmeldenummer: **94917601.0**

(22) Anmeldetag: **09.05.1994**

(51) Int Cl.[7]: **A61K 31/37**, A61K 31/425, A61K 47/30

(86) Internationale Anmeldenummer:
**PCT/EP94/01489**

(87) Internationale Veröffentlichungsnummer:
**WO 94/26267 (24.11.1994 Gazette 1994/26)**

# (54) ZUBEREITUNGEN IN FORM FESTER LÖSUNGEN

COMPOSITIONS IN THE FORM OF SOLID SOLUTIONS

COMPOSITIONS SOUS FORME DE SOLUTIONS SOLIDES

(84) Benannte Vertragsstaaten:
**AT BE CH DE DK ES FR GB GR IE IT LI LU NL PT SE**

(30) Priorität: **18.05.1993 DE 4316537**

(43) Veröffentlichungstag der Anmeldung:
**28.02.1996 Patentblatt 1996/09**

(73) Patentinhaber: **BASF Aktiengesellschaft
67063 Ludwigshafen (DE)**

(72) Erfinder:
- **GRABOWSKI, Sven
D-67061 Ludwigshafen (DE)**
- **MUELLER, Winfried
D-68163 Mannheim (DE)**
- **ROSENBERG, Joerg
D-67158 Ellerstadt (DE)**
- **BINDER, Rudolf
D-67551 Worms (DE)**
- **SANNER, Axel
D-67227 Frankenthal (DE)**

(56) Entgegenhaltungen:
**EP-A- 0 111 746**
**DE-A- 3 830 355**
**US-A- 5 073 563**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 697 867 B1

Printed by Jouve, 75001 PARIS (FR)

## Beschreibung

Die vorliegende Erfindung betrifft neue Zubereitungen in Form fester Lösungen, enthaltend

a) 1 bis 90 Gew.-% eines Cumarinderivats A aus der Gruppe der heterocyclisch substituierten Alkoxycumarine oder der mit einer Sulfonsäure veresterten Hydroxycumarine als Wirkstoff und
b) 10 bis 99 Gew.-% mindestens eines wasserlöslichen Polymeren B als Trägersubstanz.

Außerdem betrifft die Erfindung ein Verfahren zur Herstellung dieser Zubereitungen, Arzneimittel, enthaltend diese Zubereitungen, sowie deren Verwendung zur Herstellung von Arzneimitteln.
Zubereitungen, in denen der Wirkstoff homogen verteilt in einem wasserlöslichen Polymeren vorliegt, sind in zahlreichen Ausgestaltungen allgemein bekannt. Beispielsweise werden in EP-A 240 773, EP-A 462 066, EP 521 310 und Drug Development and Industrial Pharmacy, 6 (2), 137-160 (1980) Zubereitungen, enthaltend Hydroxypropylmethylcellulose bzw. Polyvinylpyrrolidon als Trägersubstanz, beschrieben.
Heterocyclisch substituierte Alkoxycumarine

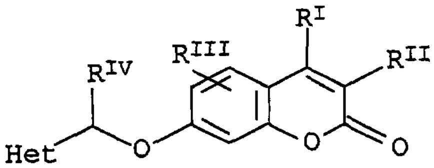

$R^I$, $R^{II}$, $R^{III}$, $R^{IV}$ = Substituenten
Het = N, S und O enthaltender heterocyclischer Rest

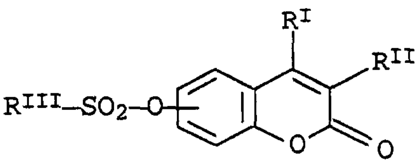

$R^I$, $R^{II}$, $R^{III}$ = Substituenten

sowie diese Substanzen als Wirkstoffe enthaltende pharmazeutische Zubereitungen sind bekannt und werden in der US-A 5 073 563 zur Behandlung von Erkrankungen des zentralen Nervensystems, insbesondere von neurodegenerativen Erkrankungen und Parkinsonismus, bzw. in der EP-B 111 746 zur Behandlung von psychischen Störungen, insbesondere von Depressionen, vorgeschlagen. Nachteil dieser Wirkstoffe und ihrer dort beschriebenen galenischen Zubereitungen ist, daß sie wegen ihrer geringen Wasserloslichkeit im Hinblick auf ihre Bioverfügbarkeit zu wunschen übrig lassen. Daher können diese in kristalliner Form vorliegenden Cumarinderivate nach Einnahme nicht vollstandig und nur sehr langsam resorbiert werden.
Der Erfindung lag daher die Aufgabe zugrunde, Zubereitungen in Form fester Lösungen mit einer guten Löslichkeic und Bioverfugbarkeit sowie einer schnellen Resorption der Cumarinderivate A zur Verfügung zu stellen.
Demgemäß wurden die eingangs definierten Zubereitungen gefunden.
Weiterhin betrifft die Erfindung ein Verfahren zu deren Herstellung, ihre Verwendung als Arzneimittel sowie ihre Darreichungsformen.
Die erfindungsgemäßen Zubereitungen enthalten

a) 1 bis 90 Gew.-%, vorzugsweise 10 bis 40 Gew.-% eines Cumarinderivats A aus der Gruppe der heterocyclisch substituierten Alkoxycumarine oder der mit einer Sulfonsäure veresterten Hydroxycumarine als Wirkstoff und

b) 10 bis 99 Gew.-%, vorzugsweise 60 bis 90 Gew.-% mindestens eines wasserlöslichen Polymeren B als Trägersubstanz.

Als heterocyclisch substituierte Alkoxycumarine A' kommen die in der US-A 5 073 563 genannten Verbindungen in Betracht, wobei hier vorzugsweise solche geeignet sind, welche einen Thiadiazol-Rest als heterocyclischen Substituenten tragen und welche in der zitierten Schrift beansprucht werden. Besonders eignen sich

- 4-Trifluormethyl-7-(2-methyl-1,3,4-thiadiazol-5-yl)-methoxycumarin

- 3,4-Dimethyl-7-(2-isopropyl-1,3,4-thiadiazol-5-yl)-methoxycumarin

- 3,6-Dichlor-4-methyl-7-(2-cyclopropylthiazol-4-yl)-methoxycumarin
- 3,4-Dimethyl-7-(2-methylthiazol-4-yl)-methoxycumarin
- 3,4-Dimethyl-7-(2-phenylthiazol-4-yl)-methoxycumarin
- 3,4-Dimethyl-7-(2-benzylthiazol-4-yl)-methoxycumarin
- 3,4-Dimethyl-7-(2-isopropylthiazol-4-yl)-methoxycumarin
- 3,4-Dimethyl-7-(2-cycloproylthiazol-4-yl)-methoxycumarin
- 3,6-Dichlor-4-methyl-7-(2-isopropylthiazol-4-yl)-methoxycumarin
- 6-Brom-3-chlor-4-methyl-7-(2-isopropylthiazol-4-yl)-methoxycumarin

Als Sulfonsäure veresterte Hydroxycumarine A" kommen die in der EP-B 111 746 beschriebenen Verbindungen in Betracht.

Ganz besonders eignet sich das erfindungsgemäße Prinzip für Zubereitungen mit 3,4-Dimethyl-7-(2-isopropyl-1,3,4-thiadiazol-5-yl)-methoxycumarin A'/1

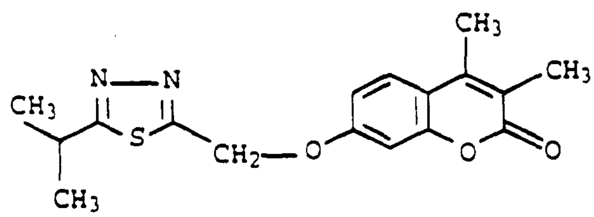

A'/1

aus der Gruppe der heterocyclisch substituierten Alkoxycumarine A' und mit 7-Hydroxy-3,4-dimethylcumarin-ethansulfonsaureester A"/1

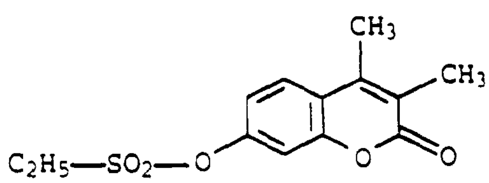

A"/1

aus der Gruppe der mit Sulfonsäure veresterten Hydroxycumarine A" als Wirkstoff.

Als wasserlösliche Polymere B seien genannt:

- Alkylcellulosen wie Methylcellulose
- Hydroxyalkylcellulosen wie Hydroxymethyl-, Hydroxyethyl-, Hydroxypropyl- und Hydroxybutylcellulose
- Hydroxyalkylalkylcellulosen wie Hydroxyethylmethyl- und Hydroxypropylmethylcellulose
- Carboxyalkylcellulosen wie Carboxymethylcellulose
- Alkalimetallsalze der Carboxyalkylcellulosen wie Natriumcarboxymethylcellulose
- Carboxyalkylcelluloseester
- N-Vinylpyrrolidon-Vinylacetat-Copolymere

- Polyvinylpyrrolidon
- Polyvinylalkohol
- Polyacrylsäure und ihre Salze
- Polymethacrylsaure und ihre Salze
- Polyalkylenoxide wie Polyethylenoxid und Polypropylenoxid sowie Copolymere aus Ethylen- und Propylenoxid
- Polysaccharide wie Alginsäure, ihre Alkali- und Ammoniaumslaze, Carrageenane, Galaktomannane, Traganth, Agar-Agar, Gummi arabicum, Guargummi und Xanthan gummi
- Chitinderivate wie Chitosan
- Pektine wie Natriumcarboxymethylamylpektin
- Stärken

sowie Gemische dieser wasserlöslichen Polymeren.

Als Polymere B sind Methylcellulosen, Hydroxypropylmethylcellulosen, Hydroxypropylcellulose, Polyvinylpyrrolidon und N-Vinylpyrrolidon-Vinylacetat-Copolymere bevorzugt, insbesondere Polyvinylpyrrolidon und Copolymere aus 40 bis 70 Gew.-% N-Vinylpyrrolidon und 30 bis 60 Gew.-% Vinylacetat. Unter wasserloslich ist zu verstehen, daß sich bei 20°C in 100 g Wasser mindestens 0,5 g, bevorzugt 2 g des Polymeren ggfs. kolloidal auflosen bzw. unter Gelbildung lösen.

Eine feste Losung liegt dann vor, wenn der Wirkstoff im wesentlichen molekulardispers in der Polymermatrix verteilt ist (J. Pharm. Sci. 60, 1281-1302, 1971).

Um die erfindungsgemäßen Zubereitungen herzustellen, kann das Cumarinderivat A entweder direkt in Form einer physikalischen Mischung mit dem Polymeren B verschmolzen werden oder mit der bereits vorliegenden Polymerschmelze gemischt werden.

Im übrigen erfolgt die Vermischung des Cumarinderivates A mit der Schmelze in an sich bekannter Weise in Extrudern, vorzugsweise in Ein- oder Doppelschneckenextrudern in einem Temperaturbereich zwischen 50 und 200°C. Die Formgebung der Cumarinderivat A haltigen Polymerschmelze zu den erfindungsgemäßen Zubereitungen kann beispielsweise durch Kalandrierung des Extrudates nach der in der EP-A 240 906 beschriebenen Methode sowie nach dem aus der DE-A 38 30 355 bekannten Verarbeitungsverfahren durch Zerkleinerung des Extrudates mit rotierenden Messern in volumengleiche - noch verformbare - Stücke mit erstarrter Oberfläche und anschließendes Verpressen zu Tabletten in den üblichen Tablettiermaschinen erfolgen.

Man kann die Vermischung des Wirkstoffes mit der Schmelze auch in anderen hierfür geeigneten Apparaturen, mit denen man üblicherweise Kunststoffe verarbeitet, z.B. Kalandern und Spritzgußformen, vornehmen.

Zur Herstellung der erfindungsgemäßen Zubereitungen kann das Cumarinderivat A auch - in einem flüchtigen Lösungsmittel aufgelöst - mit der Polymerschmelze vermischt werden. Darüber hinaus kann eine Mischung aus Cumarinderivat A und Polymer B auch durch gemeinsames Auflösen in einem flüchtigen Lösungsmittel und durch anschließendes Verdampfen des Lösungsmittels erhalten werden. Der abgekühlte Rückstand wird entsprechend der Schmelze unter Formgebung in den üblichen Apparaturen zu festen Darreichungsformen weiterverarbeitet.

In einigen Fällen kann es vor der Formgebung zweckmäßig sein, die Mischung aus A und B sowohl in Form der Schmelze als auch in Form einer Lösung z.B. auf feinverteiltem, porösem Trägermaterial wie Silicagel aufzubringen sowie Einschlußverbindungen z.B. mit Cyclodextrinen und ihren Derivaten zu bilden.

Außerdem können die erfindungsgemaßen Zubereitungen die ublichen pharmazeutischen Hilfsstoffe wie Füllstoffe, Schmiermittel, Formentrennmittel, Fließregulierungsmittel, Weichmacher, Farbstoffe und Stabilisatoren in Mengen bis zu ca. 60 Gew.-% enthalten. Diese und die im folgenden angegebenen Mengen sind jeweils bezogen auf das Gesamtgewicht der Zubereitung (= 100 %).

Als Füllstoffe seien z.B. die Oxide von Magnesium, Aluminium, Silizium und Titan sowie Lactose, Mannit, Sorbit, Xylit, Pentaerythrit und seine Derivate genannt, wobei die Menge an Füllstoff bei ca. 0,02 bis 50, vorzugsweise 0,2 bis 20 Gew.-% liegt.

Als Fließregulierungsmittel seien z.B. die Mono-, Di- und Triglyceride der langkettigen Fettsäuren wie $C_{12}$-, $C_{14}$-, $C_{16}$- und $C_{18}$-Fettsäure, Wachse wie Carnaubawachs sowie die Lecithine genannt, wobei die Menge bei ca. 0,1 bis 30, vorzugsweise 0,1 bis 5 Gew.-% liegt.

Als Weichmacher seien z.B. neben niedermolekularen Polyalkylenoxiden wie Polyethylenglykol, Polypropylenglykol und Polyethylenpropylenglykol auch mehrwertige Alkohole wie Propylenglykol, Glycerin, Pentaerythrit und Sorbit sowie Natriumdiethylsulfosuccinat, Mono-, Di- und Triacetat des Glycerin und Polyethylenglykolstearinsäureester genannt. Dabei liegt die Menge an Weichmacher bei ca. 0,5 bis 15, vorzugsweise 0,5 bis 5 Gew.-%.

Als Schmiermittel seien z.B. Stearate von Aluminium oder Calcium sowie Talkum und Silikone genannt, wobei ihre Menge bei ca. 0,1 bis 5, vorzugsweise 0,1 bis 3 Gew.-% liegt.

Als Stabilisatoren seien beispielsweise Lichtstabilisatoren, Antioxidantien, Radikalfänger und Stabilisatoren gegen mikrobiellen Befall genannt, wobei ihre Menge vorzugsweise bei ca. 0,01 bis 0,05 Gew.-% liegt.

Es ist möglich, die Hilfsstoffe in die Schmelze oder Lösung aus Cumarinderivat A und Polymer B zu mischen. Ferner können die Hilfsstoffe zusammen mit dem Cumarinderivat A in die Polymerschmelze oder in die Lösung von Polymer B eingearbeitet werden. Außerdem können Gemische aus Hilfsstoffen, dem Cumarinderivat A und dem Polymer B direkt verschmolzen werden oder gemeinsam in einem Lösungsmittel gelöst werden. Im allgemeinen ist es üblich, eine physikalische Mischung aus Hilfsstoffen, Cumarinderivat A und Polymer B gemeinsam zu verschmelzen.

Die erfindungsgemäßen Zubereitungen werden als Arzneimittel verwendet und in Form von Pulvern, Granulaten, Tabletten, Pellets, Zäpfchen oder in Kapseln eingesetzt.

Für die orale Verabreichung empfiehlt es sich, die Zubereitungen in bekannter Weise mit Farbüberzugen z. B aus Titandioxid und aus Buntpigmenten zur Verbesserung des Aussehens zu überziehen. Zur Geschmacksverbesserung eignen sich Überzüge, beispielsweise Glucose, Saccharose, Xylit und Mannit.

Die neuen Zubereitungen in Form fester Lösungen weisen gegenüber dem Stand der Technik Vorteile auf, indem das Cumarinderivat A besser freigesetzt wird, wodurch seine Bioverfügbarkeit und Resorption erheblich gesteigert werden. Durch die nahezu homogene Verteilung des amorphen Cumarinderivates A in der polymeren Trägersubstanz B wird eine Verbesserung der Löslichkeit gegenüber von kristallinem Cumarinderivat A erreicht.

Beispiele 1 bis 9

Für die Herstellung der erfindungsgemäßen Zubereitungen wurden die Wirkstoffe

A'/1 3,4-Dimethyl-7-(2-isopropyl-1,3,4-thiadiazol-5-yl)-methoxycumarin und

A"/1 7-Hydroxy-3,4-dimethylcumarin-ethansulfonsaureester

verwendet. Ihre Synthese erfolgte nach den in US-A 5 073 563 bzw. EP-B 111 746 beschriebenen Verfahren.

Folgende kommerziell erhältlichen Polymere B wurden verwendet: (Relative Viskositäten bestimmt nach der Kapillarmethode ASTM D 2365-72 (Europäisches Arzneibuch, Bd. III., S. 37))

B/1 Copolymerisat aus 60 Gew.-% N-Vinylpyrrolidon (NVP) und 40 Gew.-% Vinylacetat; V = 1,18-1,31 cps (1 % Lösung in Wasser, 25°C) (Kollidon® VA64 der Firma BASF AG)

B/2 Polyvinylpyrrolidon; V = 1,430-1,585 cps (5 % Lösung in Wasser, 25°C) (Kollidon® 17PF der Firma BASF AG)

B/3 Polyvinylpyrrolidon; V = 1,201-1,276 cps (1 % Lösung in Wasser, 25°C) (Kollidon® 30 der Firma BASF AG)

(V = Viskosität)

Als Hilfsstoffe C wurden folgende Substanzen verwendet:

C/1 Polyethylenoxid, $\overline{M}_w$ = 6000 (Lutrol® E6000 der Firma BASF AG)

C/2 Polyethylenoxid, $\overline{M}_w$ = 1500 (Lutrol® E1500 der Firma BASF AG)

C/3 Lactose-Monohydrat, feingepulvert; nach DAB, Ph Eur, BP, USP (Firma Meggle)

($\overline{M}_w$ = mittleres Molekulargewicht, Gewichtsmittel)

Die in den Beispielen 1 bis 6 und 9 angegebenen Mengen an Cumarinderivat A, an Polymer B und zum Teil an Hilfsstoff C wurden gemischt, anschließend in einem Doppelschneckenextruder eingebracht und bei über 5 Temperaturzonen von 60 bis 130°C extrudiert. Der austretende Polymerstrang wurde in einem Kalander miteinander gegenüberliegenden, nach innen gewölbten Vertiefungen in den Walzenmänteln eingeführt und zu 1000 mg-Tabletten form-geprägt. Es wurden hellgelbe, durchsichtige Tabletten erhalten.

In den Beispielen 1 bis 6 wurde die Wirkstoffreisetzung mittels der Rührflügelmethode (paddle-Methode nach USP XXI, US-Arzneibuch) gemessen. Diese In-Vitro-Prüfungsmethode dient zur Bestimmung der Lösungsrate von

wirkstoffhaltigen Formlingen (z.B. Tabletten).

Hierzu wurden 900 ml einer 0,1 N Salzsäure mit 0,05 mol/l Natriumlaurylsulfat in einem 1 1-Gefäß mit Rundboden auf 37°C temperiert. Während des Prüfvorganges befand sich die zu prüfende 1000 mg-Tablette auf dem Rundboden des Gefäßes zentral unter dem Rührflügel, der sich mit einer Drehzahl von 100 U/min bewegte. Nach einer Versuchsdauer von jeweils 1 Stunde wurde die Menge an freigesetzten Wirkstoff UV-spektroskopisch bestimmt.

In den Beispielen 7 bis 9 wurde mittels einer In-Vivo-Prüfungsmethode die absolute Bioverfügbarkeit F ermittelt.

Unter der Bioverfügbarkeit (biologischen Verfügbarkeit; F) eines Arzneimittels versteht man Geschwindigkeit und Ausmaß, womit ein therapeutisch wirksamer Bestandteil aus einer Arzneiform freigesetzt, resorbiert und schließlich am Wirkort verfügbar wird. Bei intravenöser Applikation beträgt die Bioverfügbarkeit 100 %.

Die absolute Bioverfugbarkeit F wird nach folgender Gleichung ermittelt:

$$F = \frac{AUC_x \cdot \text{Dosis i.v.}}{AUX_{i.v.} \cdot \text{Dosis}_x} \cdot 100\ \%$$

AUC = area under the curve; Fläche unter der Konzentration-Zeit-Kurve (Plasmaspiegelkurve)
$AUC_x$ = Fläche unter der Kurve bei beliebiger Applikation
$AUC_{i.v.}$ = Fläche unter der Kurve bei intravenöser Applikation

Bei der In-Vivo-Prüfungsmethode wurde jeweils einmalig 1 Tablette mit 300 mg Wirkstoff pro Hund (Beagle) verabreicht. Über einen Zeitraum von 24 Stunden wurden Blutproben in einem definierten Zeitraster entnommen und die Konzentration des Wirkstoffes im Blut bestimmt.

Die Einzelheiten dieser Versuche sowie deren Ergebnisse sind der Tabelle 1 zu entnehmen.

Tabelle 1

| Beispiel | Cumarinderivat A Gew.-% | | Polymer B Gew.-% | | Hilfsstoff C Gew.-% | | Wirkstoffreisetzung nach 1 h** (%) | absolute Bioverfügbarkeit F (%) |
|---|---|---|---|---|---|---|---|---|
| 1 | 20 | A"/1 | 80 | B/1 | - | | 6 | nicht untersucht |
| 2 | 20 | A"/1 | 80 | B/2 | - | | 77 | |
| 3 | 20 | A"/1 | 60 | B/1 | 20 | C/1 | 37 | |
| 4 | 20 | A"/1 | 60 | B/2 | 20 | C/3 | 78 | |
| 5 | 19,5 | A"/1 | 40,5 | B/1 | 40 | C/3 | 22 | |
| 6 | 20 | A"/1 | 40 | B/2 | 40 | C/3 | 44 | |
| 7 (a)* | 50 | A'/1 | - | | 50 | C/3 | - | 1,00 (c) |
| 8 (b)* | 10 | A'/1 | - | | 90 | C/2 | - | 0,17 (d) |
| 9 | 10 | A'/1 | 90 | B/1 | - | | - | 3,05 (d) |

a) Verreibung von A'/1 mit Lactose (Vergleichsbeispiel)
b) Schmelzeinbettung (Vergleichsbeispiel)
c) F bestimmt an einem Hund
d) Mittelwert aus F, bestimmt an 4 Hunden (berechnet über Median)
* Vergleichsbeispiel
** Alle Formen erreichen eine Wirkstoff-Freisetzung von 100 % nach spätestens 8 h.

Beispiel 10

In einem weiteren Beispiel wurde die Bioverfügbarkeit von A"/1 in den erfindungsgemaßen Zusammensetzungen gemessen. Dazu wurde die Pharmakokinetik einer Extrudatzusammensetzung (feste Losung, Wirkstoff liegt amorph vor) aus Hund (Beagle) getestet. Die erzielten Plasmaspiegel sind in der Tabelle 2 angegeben. Zum Vergleich wurden auch die mit einem Granulat erreichten werte (Wirkstoff liegt vermahlen, aber kristallin vor) ermittelt.

| Zusammensetzung Extrudat (Tablette, ca. 1 g): | |
|---|---|
| A"/1 | 18,87 Gew.-% |
| Vinylpyrrolidon-Vinylacetat-Copolymer (60:40), (Kollidon® VA-64) | 41,13 Gew.-% |
| Laktose | 40,00 Gew.-% |
| **Zusammensetzung Granulat (Kapseln, Größe 0) :** | |
| A"/1 | 77,55 Gew.-% |
| Laktose | 11,64 Gew.-% |
| Mikrokrist. Cellulose | 7,75 Gew.-% |
| Polyvinylpyrrolidon, K-Wert 30 | 2,58 Gew.-% |
| Mg-Stearat | 0,48 Gew.-% |

Die Ergebnisse sind der Tabelle 2 zu entnehmen.

Tabelle 2

Plasmaspiegel von A"/1 (Esupron) bei Beagle-Hunden - 400 mg/kg KG Esupron p.o. pro Tag uber 14 Tage

| | Zeit nach Applikation [h] | Tier Nr. 3 400 mg/kg KG Granulat [ng/ml] | Tier Nr. 4 400 mg/kg KG Granulat [ng/ml] | Tier Nr. 5 400 mg/kg KG Extrudat [ng/ml] | Tier Nr. 6 400 mg/kg KG Extrudat [ng/ml] |
|---|---|---|---|---|---|
| 1. Applikation | vor Substanzgabe | *) | 2.68 | 11.38 | 1.73 |
| | 1 | 28.56 | 1.72 | 1245.92 | 2558.33 |
| | 2 | 10.64 | 2.88 | 6907.77 | 8097.63 |
| | 3 | 11.52 | 2.62 | 7307.85 | 12625.00 |
| | 4 | 7.81 | 1.81 | 4653.01 | 5178.90 |
| | 6 | 4.18 | 1.14 | 1011.11 | 1497.21 |
| | 8 | 2.70 | 0.78 | 339.09 | 776.71 |
| | 24 | 9.17 | 3.29 | 14.13 | 53.98 |
| 13. Applikation | 24 | 7.16 | 9.31 | 17.87 | 54.34 |
| 14. Applikation | 1 | 6.76 | 9.97 | 178.59 | 4795.10 |
| | 2 | 16.48 | 0.66 | 263.98 | 12257.10 |
| | 3 | 7.45 | 9.42 | 461.53 | 6466.05 |
| | 4 | 4.70 | 7.15 | 542.59 | 3096.27 |
| | 6 | 5.22 | 6.66 | 3503.58 | 1086.55 |
| | 8 | 7.56 | 5.99 | 1242.95 | 714.03 |
| | 24 | 1.63 | 3.33 | 7.48 | 36.62 |

*) Wert unterhalb der Nachweisgrenze von 0,2 [ng/ml]

## Patentansprüche

1. Zubereitungen in Form fester Lösungen, enthaltend

   a) 1 bis 90 Gew.-% eines Cumarinderivats A aus der Gruppe der heterocyclisch substituierten Alkoxycumarine oder der mit einer Sulfonsäure veresterten Hydroxycumarine als Wirkstoff und

   b) 10 bis 99 Gew.-% mindestens eines wasserlöslichen Polymeren B als Trägersubstanz.

2. Zubereitungen nach Anspruch 1, enthaltend

   a) 10 bis 40 Gew.-% des Cumarinderivat A und

   b) 60 bis 90 Gew.-% des (der) wasserlöslichen Polymeren B.

3. Zubereitungen nach Anspruch 1 oder 2, enthaltend 3,4-Dimethyl-7-(2-isopropyl-1,3,4-thiadiazol-5-yl)-methoxycumarin oder 7-Hydroxy-3,4-dimethylcumarinethansulfonsäureester als Cumarinderivat A.

4. Zubereitungen nach den Ansprüchen 1 bis 3, enthaltend Polyvinylpyrrolidon und/oder N-Vinylpyrrolidon-Vinylacetat-Copolymere als Polymeren B.

5. Verfahren zur Herstellung der Zubereitungen gemäß den Ansprüchen 1 bis 4, **dadurch gekennzeichnet, daß** man das Cumarinderivat A mit der Schmelze oder mit einer Lösung des Polymeren B vermischt und die Schmelze oder den - nach Entfernen des Lösungsmittels - verbleibenden Rückstand unter Formgebung zu Teilchen weiterverarbeitet.

6. Verwendung der Zubereitungen gemäß den Ansprüchen 1 bis 4 zur Herstellung von Arzneimitteln.

7. Pulver, Granulate, Tabletten, Pellets, Zäpfchen und Injektionslösungen aus den Zubereitungen gemäß den Ansprüchen 1 bis 4.

8. Arzneimittel, enthaltend Zubereitungen gemäß Anspruch 1.

## Claims

1. A composition in the form of a solid solution containing

   a) 1-90% by weight of a coumarin derivative A from the group of alkoxycoumarins with a heterocyclic substituent or hydroxycoumarins esterified with a sulfonic acid as active substance and

   b) 10-99% by weight of at least one water-soluble polymer B as carrier substance.

2. A composition as claimed in claim 1, containing

   a) 10-40% by weight of the coumarin derivative A and

   b) 60-90% by weight of the water-soluble polymer(s) B.

3. A composition as claimed in claim 1 or 2, containing 3,4-dimethyl-7-(2-isopropyl-1,3,4-thiadiazol-5-yl)methoxycoumarin or 7-hydroxy-3,4-dimethylcoumarin ethanesulfonic ester as coumarin derivative A.

4. A composition as claimed in any of claims 1 to 3, containing polyvinylpyrrolidone and/or N-vinylpyrrolidone/vinyl acetate copolymers as polymer B.

5. A process for producing a composition as claimed in any of claims 1 to 4, which comprises mixing the coumarin derivative A with the melt or with a solution of polymer B and further processing the melt or the residue remaining after removal of the solvent, with shaping to particles.

6. The use of a composition as claimed in any of claims 1 to 4 for producing a drug.

7. Powders, granules, tablets, pellets, suppositories and injection solutions comprising a composition as claimed in any of claims 1 to 4.

8. A drug containing compositions as claimed in claim 1.

**Revendications**

1. Compositions à l'état de solutions solides, contenant

   a) 1 à 90 % en poids d'un dérivé de la coumarine A appartenant au groupe des alcoxycoumarines à substituants hétérocycliques ou des hydroxycoumarines estérifiées par un acide sulfonique, constituant la substance active et

   b) 10 à 99 % en poids d'au moins un polymère soluble dans l'eau B qui constitue la substance véhicule.

2. Compositions selon la revendication 1, contenant

   a) 10 à 40 % en poids du dérivé de la coumarine A et

   b) 60 à 90 % en poids du ou des polymères solubles dans l'eau B.

3. Compositions selon l'une des revendications 1 ou 2 contenant, en tant que dérivé de la coumarine A, la 3,4-diméthyl-7-(2-isopropyl-1,3,4-thiadiazol-5-yl)-méthoxy-coumarine ou l'ester méthanesulfonique de la 7-hydroxy-3,4-diméthyl-coumarine.

4. Compositions selon les revendications 1 à 3 contenant, en tant que polymère B, une polyvinylpyrrolidone et/ou un copolymère N-vinylpyrrolidone-acétate de vinyle.

5. Procédé de préparation des compositions selon les revendications 1 à 4, **caractérisé par le fait que** l'on mélange le dérivé de la coumarine A avec la masse fondue ou avec une solution du polymère B et on met la masse ou bien le résidu obtenu après élimination du solvant à l'état de particules par façonnage.

6. Utilisation des compositions selon les revendications 1 à 4 pour la préparation de médicaments.

7. Poudres, granulés, comprimés, pilules, cachets et solutions pour injections consistant en les compositions selon les revendications 1 à 4.

8. Médicaments contenant des compositions selon la revendication 1.